# EUROPEAN PATENT SPECIFICATION

(11) **EP 1 465 617 B1**
(45) Date of publication and mention of the grant of the patent: **15.10.2008**
(21) Application number: 02799240.3
(22) Date of filing: 12.12.2002
(51) Int. Cl.: A61K 31/33, A61P 25/00

(54) **PROCESS FOR AFFECTING NEUROLOGIC PROGRESSION**
VERFAHREN ZUR BEEINFLUSSUNG NEUROLOGISCHER PROGRESSION
PROCEDE PERMETTANT D'AFFECTER LA PROGRESSION NEUROLOGIQUE

(30) Priority: 13.12.2001 US 339650 P; 07.01.2002 US 346584 P; 30.01.2002 US 353090 P
(43) Date of publication of application: 13.10.2004
(73) Proprietor: PHARMACYCLICS, INC., Sunnyvale, CA 94085-4521 (US)
(72) Inventor: MILLER, Richard, A., Portola Valley, CA 94028 (US)
(74) Representative: Glawe, Delfs, Moll
(86) International application number: PCT/US2002/039974
(87) International publication number: WO 2003/049731

(56) References cited:
- US-A- 5 569 759
- JULIETTE VIALA AND ALL.: "Phase IB and II multidose trial of Gadolinium Texaphyrin, a radiation sensitizer detectable at MR imaging: preliminary results in brain metastases" RADIOLOGY, vol. 212, 1999, pages 755-759, XP002341426
- DAVID I. ROSENTHAL AND ALL.: "A phase I single-dose trial of Gadolinium Texaphyrin (Gd-Tex), a tumor selective radiation sensitizer detectable by magnetic resonance imaging" CLINICAL CANCER RESEARCH, vol. 5, April 1999 (1999-04), pages 739-745, XP002341427
- ERIC J. BERNHARD AND ALL.: "Re-evaluating Gadolinium(III) Texaphyrin as a radiosensitizing agent" CANCER RESEARCH, vol. 60, January 2000 (2000-01), pages 86-91, XP002341428

## Description

### CLAIM OF PRIORITY

The present Application claims priority from **PCT International Patent Application No.** PCT/US02/39974, filed December 12, 2002**,** which claims the benefit of priority from U.S. Provisional Patent Application No. 60/339,650, filed December 13, 2001, U.S. Provisional Patent Application No. 60/346,584, filed January 7, 2002, and U.S. Provisional Patent Application No. 60/353,090, filed January 30, 2002**,** the contents of which are incorporated herein by reference in their entirety.

### FIELD OF INVENTION

The present invention relates to use of a compound of Formula I for improving neurologic functions in patients afflicted with systemic lung cancer that has metastasized to the brain (brain metastases).

### BACKGROUND OF THE INVENTION

Brain metastases are known to be a secondary manifestation of some forms of systemic cancer, including breast cancer, lung cancer, pancreatic cancer, skin cancer, and prostrate cancer. The effect of radiation therapy in the treatment of brain metastases has been studied over the years. One such study was reported by Chao-et al., Cancer, pp. 682-89 (1954). A number of factors have been reported to influence the degree of response to irradiation. Those factors include primary site and extent of metastases, status of primary site, neurologic status, and general functional status.

With the above in mind, the Radiation Therapy Oncology Group conducted two randomized national Phase III trials. Results of these trials were reported by Borgelt et al., International Journal of Radiation Oncology Biology Physics, Vol. 6, pp. 1-8. Among the findings, Borgelt et al. report that primary site of the underlying cancer, such as lung cancer, breast cancer, etc., had no influence on response in the neurologic progression category. Similarly, the status of primary tumor and chemotherapy status had no influence in time to neurologic progression. It was found that time to neurologic progression was longer for ambulatory patients and, in general, for patients with brain as the only site of metastases. Patients with breast cancer as the primary cancer were also found to have longer time to neurologic progression, probably because these patients survived longer. Another end point reported by Borgelt et al. was survival in patients with brain metastases. Looking at survival as the end point the study indicated that ambulatory patients survived longer (21 weeks median) than non-ambulatory patients (12 weeks). It is further reported that breast cancer patients, as a group, survived longer (21 weeks) than lung cancer patients (16 weeks).

Additionally, Viala et al., Radiology, Vol. 212, No. 3, pp. 755-759 (September, 1999); and Carde et al., Journal of Clinical Oncology, Vol. 19, No. 7, pp. 2074.2083 (April, 2001) reported that the compound of Formula I improved neurological function in patients afflicted with brain metastases and having breast cancer, lung cancer, melanoma and other cancers as primary cancers.

### SUMMARY OF THE INVENTION

Applicant has surprisingly/unexpectedly discovered that the compound of Formula I does not significantly improve neurological function in patients afflicted with brain metastases and a broad range of systemic cancers, as previously reported. Rather, the compound of Formula I has been found to improve neurological function selectively in patients afflicted with lung cancer wherein the lung cancer has metastasized to the brain (brain metastases).

Accordingly, the present invention provides a method for delaying or slowing neurologic progression selectively in a human afflicted with lung cancer, comprising administering to the human an effective amount of a compound of Formula I:

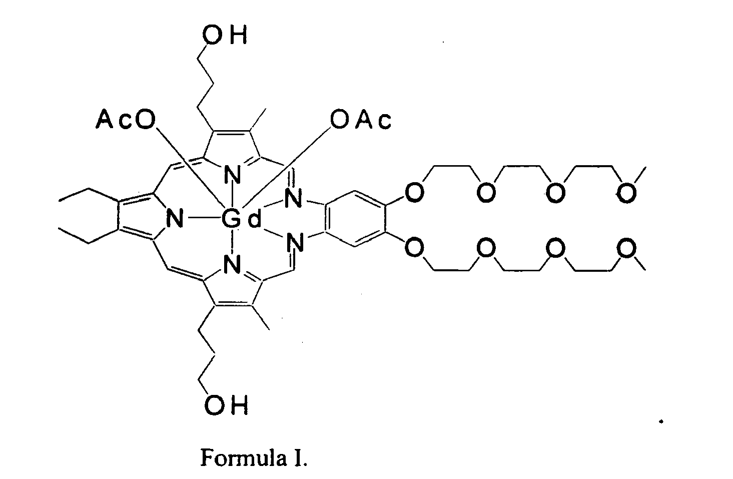

The invention also provides a method for prolonging time to neurologic progression selectively in a human afflicted with lung cancer, comprising administering to the human an effective amount of a compound of Formula I.

The invention also provides a method for prolonging time to deterioration of memory function selectively in a human afflicted with lung cancer, comprising administering to the human an effective amount of a compound of Formula I.

The invention also provides a method for prolonging time to deterioration executive function selectively in a human afflicted with lung cancer, comprising administering to the human an effective amount of a compound of Formula I. Yet another aspect of the present invention provides a method of prolonging survival time selectively in humans afflicted with lung cancer and brain metastases, comprising administering to the humans an effective amount of a compound of Formula I.

The present invention provides use of a compound of Formula I to prepare a medicament useful for delaying/slowing neurologic progression selectively in a human afflicted with lung cancer and brain metastases. Also provided by the present invention is the use of a compound of Formula I to prepare a medicament useful for prolonging/improving time to neurologic progression selectively in a human afflicted with lung cancer and brain metastases.

Another aspect of the present invention provides the use of a compound of Formula I to prepare a medicament useful for prolonging time to deterioration of memory function selectively in a human afflicted with lung canter and brain metastases. Another embodiment of the present invention provides the use of a compound of Formula I to prepare a medicament useful for prolonging time to deterioration of executive function selectively in a human afflicted with lung cancer and brain metastases. Yet another embodiment of the present invention provides the use of a compound of Formula I to prepare a medicament useful for prolonging/improving survival time selectively in a human afflicted with lung cancer and brain metastases.

Another aspect of the present invention provides all the above uses wherein the medicament is in the form of an injectable composition comprising a pharmaceutically acceptable carrier.

### DETAILED DESCRIPTION OF THE INVENTION

As used herein, the following terms have the given meaning unless otherwise specifically noted.

The term "neurological progression" includes a change (going from better to worse, i.e., worsening) in clinically detectable signs and symptoms controlled by the central nervous system (e.g., neuromuscular, central, peripheral, autonomic and the like).

The term "memory function" includes the ability of a patient to retain information. The term "executive function" includes the ability of a patient to recognize patterns and demonstrate abilities to plan and organize.

The term "p-value" describes the probability an event is attributed to random chance (e.g., p = 0.05 is 5% probability result is attributed to random chance - 95% probability it is not attributed to random chance).

The term "statistically significant" means that an observation or an event is not attributed to random chance, p = 0.05 or better.

As described herein, applicant has discovered that the compound of Formula I provides certain specific beneficial effects for a host afflicted with lung cancer wherein the lung cancer has metastasized (spread) to the hosts brain (brain metastases). It is thus understood that the present invention relates to methods of treating, improving, delaying, prolonging and the like, a host afflicted with lung cancer wherein the lung cancer has metastasized (spread) to the hosts brain (brain metastases). Contrary to previous reports, the compound of Formula I does not provide similar beneficial effects for humans suffering from other forms of cancer. Accordingly, the term "selectively" means that the beneficial effects of the compound of Formula I are realized by a human having lung cancer. Although the human may have other cancers in addition to lung cancer, according to the methods of the invention, the compound of Formula I only produces beneficial effects that are associated with the lung cancer.

The terms neurological progression, memory function, and executive function, are well known in the relevant field; assays and protocols for measuring these parameters are also well known, as reported by Mehta et al., in Journal of Clinical Oncology, Vol. 20, No. 16, pp. 3445-3453 (August 15,2002).

The following specific values illustrate representative embodiments of the invention but they are not intended to be limiting.

Specifically, the compound can be administered in the form of an injectable composition comprising a pharmaceutically acceptable carrier.

Specifically, the methods of the invention are useful for treating humans with lung cancer that has metastasized to the human's brain prior to administration of the compound.

Specifically, the methods of the invention are useful for treating humans with brain metastases pursuant to a primary tumor that have not been subject to another cancer therapy.

The compound of Formula I can be formulated as a pharmaceutical compositions and administered to a mammalian host, such as a human patient in a variety of forms adapted to the chosen route of administration, that is, orally or parenterally, by intravenous, intramuscular, topical or subcutaneous routes.

The active compound may also be administered intravenously or intraperitoneally by infusion or injection. Solutions of the active compound or its formulation can be prepared in water, optionally mixed with a nontoxic surfactant. Dispersions can also be prepared in glycerol, liquid polyethylene glycols, triacetin, and mixtures thereof and in oils. Under ordinary conditions of storage and use, these preparations contain a preservative to prevent the growth of microorganisms. An example of formulating the active compound is as described in U.S. Patent No. 6,069,140.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician.

In general a suitable dose, for example, will be in the range of from about 5 mg/kg to about 10 mg/kg per day for 10 days. The dosing need not be done on 10 consecutive days.

The compound of Formula I can be used to selectively slow neurologic progression in a human afflicted with brain metastases from lung cancer. Specifically, the compound of Formula I can improve the overall time to neurologic progression, memory function progression, executive function progression, and/or decrease death with evidence of neurologic progression.

The invention will now be illustrated by the following non-limiting Examples.

### Examples

The compound of Formula I can be prepared as outlined in U.S. Patent No. 5,569,759.

### Example 1

Patients with brain metastases were treated by administering the compound of Formula I in combination with Whole Brain Radiation Therapy (WBRT) (this patient group is hereafter referred to as Experimental Group). It was found that overall time to Neurologic Progression, as observed and reported by clinical investigators, improved significantly in patients with lung cancer as the primary cancer. The results are tabulated below.

**Table I: Time to Neurologic Progression**

| **Underlying cancer** | **WBRT only** | **WBRT + MGd** | **p Value** |
|---|---|---|---|
| Lung cancer | 3.7 mos | 5.5 mos | 0.025 |
| Non-Lung cancer | 4.3 mos | 4.1 mos | 0.98 |
| All cancers | 3.8 mos | 4.3 mos | 0.04 |
| (including lung cancer) | | | |

Control in the above table (and also as hereinafter referred to) is the group of patients who are only administered WBRT. Compound of Formula I is represented by MGd in the above table. Non-lung cancer included breast cancer, melanoma and other cancers.

### Example 2

Neurocognitive function was studied by evaluating Memory and Executive Function tests as reported by Mehta et al., in Journal of Clinical Oncology, Vol. 20, No. 16, pp. 3445-3453 (August 15, 2002). The Memory test improvement in the Experimental Group of patients as opposed to the Control group is shown below:

**Table II: Memory Function plus Executive Function (Hazard ratio)**

| **Underlying cancer** | **WBRT only** | **WBRT + MGd** | **p Value** |
|---|---|---|---|
| Lung cancer | 1 | 1.79 | 0.0626 |
| Non-Lung cancer | 1 | 0.65 | 0.2653 |
| All cancers | 1 | 1.18 | 0.4962 |
| (including lung cancer) | | | |

The Experimental Group showed a statistically significant effect in Lung Cancer patients across Memory and Executive function (p = 0.0626; Hazard Ratio 1.79). A hazard ratio of 1.79 indicates that the Memory and Executive Function in lung cancer patients afflicted with brain metastases lasted (or not adversely affected due to the brain metastases) 1.79 times (or 79%) longer than in patients who only received WBRT. Memory and Executive Function in non-lung cancer patients afflicted with brain metastases lasted (or not adversely affected due to the brain metastases) 0.65 times (or 35%) less than in patients who only received WBRT. Additionally, as a whole, Memory and Executive Function in lung and non-lung cancer patients afflicted with brain metastases lasted (or not adversely affected due to the brain metastases) 1.18 times (or 18%) longer than in patients who only received WBRT.

The above data clearly indicates that patents with lung cancer and brain metastases clearly exclusively benefit from WBRT and compound of Formula I.

The following table shows the effect of WBRT alone and with MGd on the Executive function:

**Table III: Executive Function (Hazard ratio)**

| **Underlying cancer** | **WBRT only** | **WBRT + MGd** | **p Value** |
|---|---|---|---|
| Lung cancer | 1 | 1.67 | 0.0806 |
| Non-Lung cancer | 1 | 0.62 | 0.2064 |
| All cancers | 1 | 1.12 | 0.5948 |
| (including lung cancer) | | | |

The Experimental Group showed a statistically significant effect in Lung Cancer patients across Executive function (p = 0.0806; Hazard Ratio 1.67). A hazard ratio of 1.67 indicates that the Executive Function in lung cancer patients afflicted with brain metastases lasted (or not adversely affected due to the brain metastases) 1.67 times (or 67 %) longer than in patients who only received WBRT. Executive Function in non-lung cancer patients afflicted with brain metastases lasted (or not adversely affected due to the brain metastases) 0.62 times (or 38%) less than in patients who only received WBRT. Additionally, as a whole, Executive Function in lung and non-lung cancer patients afflicted with brain metastases lasted (or not adversely affected due to the brain metastases) 1.12 times (or 12 %) longer than in patients who only received WBRT.

The above data clearly indicates that patents with lung cancer and brain metastases clearly exclusively benefit from WBRT and compound of Formula I.

The following table shows the effect of WBRT alone and with MGd on the Executive function:

**Table IV: Memory Function (Hazard ratio)**

| **Underlying cancer** | **WBRT only** | **WBRT + MGd** | **p Value** |
|---|---|---|---|
| Lung cancer | 1 | 1.92 | 0.0717 |
| Non-Lung cancer | 1 | 0.69 | 0.3810 |
| All cancers | 1 | 1.23 | 0.4343 |
| (including lung cancer) | | | |

The Experimental Group showed a statistically significant effect in Lung Cancer patients across Memory function (p = 0.0717; Hazard Ratio 1.92). A hazard ratio of 1.92 indicates that the Memory Function in lung cancer patients afflicted with brain metastases lasted (or not adversely affected due to the brain metastases) 1.92 times (or 92%) longer than in patients who only received WBRT. Memory Function in non-lung cancer patients afflicted with brain metastases lasted (or not adversely affected due to the brain metastases) 0.69 times (or 31 %) less than in patients who only received WBRT. Additionally, as a whole, Memory Function in lung and non-lung cancer patients afflicted with brain metastases lasted (or not adversely affected due to the brain metastases) 1.23 times (or 23 %) longer than in patients who only received WBRT.

The above data clearly indicates that patents with lung cancer and brain metastases clearly exclusively benefit from WBRT and compound of Formula I.

Neurocognitive tests comprised, (1) 3 Memory tests (Hopkins Verbal Learning Test): the Immediate Recall test, the Delayed Recall test, and the Recognition test; (2) three Executive Function Tests: COWA. Trail making Test A, and Trail Making Test B; and (3) two Fine Motor Tests: Pegboard Dominant Hand, and Pegboard Non-dominant hand. These results are reported by Mehta et al., in Journal of Clinical Oncology, Vol. 20, No. 16, pp. 3445-3453 (August 15, 2002).

The surprising and unexpected benefit in Experimental Group of Lung Cancer patients is summarized below:

The above results demonstrate that the compound of Formula I is effective in slowing or delaying neurologic progression in a host (such as a human) afflicted with lung cancer which has metastasized to the brain (brain metastasis). The results also demonstrate that the compound of Formula I delays/improves the overall time to neurologic progression, prolonging time to deterioration of memory function, executive function, and/or improving/prolonging survival time in lung cancer patients. The selective effects towards lung cancer-brain metastases over other cancers that have metastasized to the brain are surprising. It is understood that methods of the present invention are superior to treating a host, afflicted with lung cancer that has metastasized to the brain (brain metastasis), with whole brain radiation therapy (WBRT) or not treating the host with WBRT.

## Claims

1. Use of a compound of Formula I 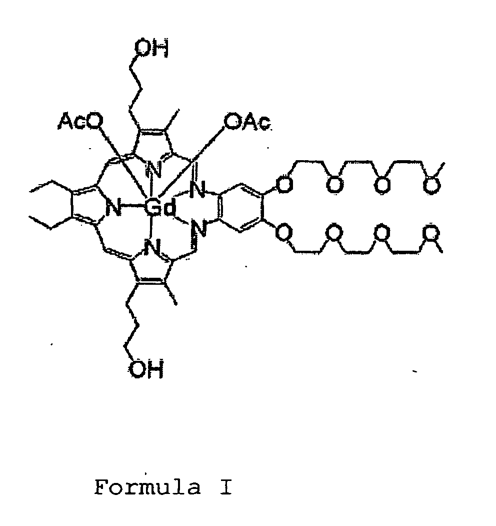 to prepare a medicament useful for delaying/slowing neurologic progression selectively in a human afflicted with lung cancer and brain metastases of said lung cancer.

2. Use of a compound of Formula 1 to prepare a medicament useful for prolonging/improving time to neurologic progression selectively in a human afflicted with lung cancer and brain metastases of said lung cancer.

3. Use of a compound of Formula I to prepare a medicament useful for prolonging time to deterioration of memory function selectively in a human afflicted with lung cancer and brain metastases of said lung cancer.

4. Use of a compound of Formula I to prepare a medicament useful for prolonging time to deterioration of executive function selectively in a human afflicted with lung cancer and brain metastases of said lung cancer.

5. The use of any of the claims 1 to 4, wherein the medicament is in the form of an injectable composition comprising a pharmaceutically acceptable carrier.

6. The use of any of the claims 1 to 5, wherein the lung cancer has metastasized to the brain of the human.

7. The use of claim 6, wherein the human is further provided radiation therapy.

8. The use of claim 7, wherein the radiation therapy is whole brain radiation therapy.

## Patentansprüche

1. Verwendung einer Verbindung der Formel I 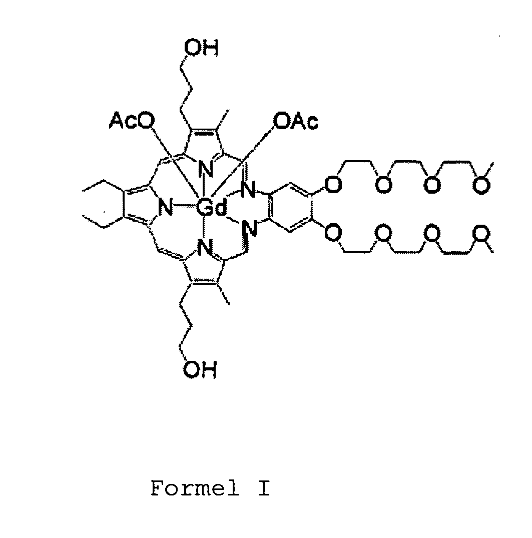 zur Herstellung eines Medikaments, das nützlich ist die neurologische Progression selektiv bei einem Menschen der an Lungenkrebs und Gehirn-Metastasen des besagten Lungenkrebses leidet zu verzögern/ zu verlangsamen.

2. Verwendung einer Verbindung der Formel I zur Herstellung eines Medikaments, das nützlich ist die Zeit bis zur neurologischen Progression selektiv bei einem Menschen der an Lungenkrebs und Gehirn-Metastasen des besagten Lungenkrebses leidet zu verlängern/ zu verbessern.

3. Verwendung einer Verbindung der Formel I zur Herstellung eines Medikament, das nützlich ist die Zeit bis zum Verfall der Gedächtnisfunktion selektiv bei einem Menschen der an Lungenkrebs und Gehirn-Metastasen des besagten Lungenkrebses leidet zu verlängern.

4. Verwendung einer Verbindung der Formel I zur Herstellung eines Medikaments, das nützlich ist die Zeit bis zum Verfall der Exekutivfunktion selektiv bei einem Menschen der an Lungenkrebs und Gehirn-Metastasen des besagten Lungenkrebses leidet zu verlängern.

5. Verwendung nach einem der Ansprüche 1 bis 4, **dadurch gekennzeichnet, dass** das Medikament in Form einer injizierbaren Verbindung ist, umfassend einen pharmazeutisch annehmbaren Träger.

6. Verwendung nach einem der Ansprüche 1 bis 5, **dadurch gekennzeichnet, dass** der Lungenkrebs in das Gehirn des Menschen metastasiert ist.

7. Verwendung nach Anspruch 6, **dadurch gekennzeichnet, dass** der Mensch des weiteren einer Strahlentherapie unterzogen wird.

8. Verwendung nach Anspruch 7, **dadurch gekennzeichnet, dass** die Strahlentherapie eine Gesamtgehirnstrahlentherapie ist.

## Revendications

1. Utilisation d'un composé de formule I 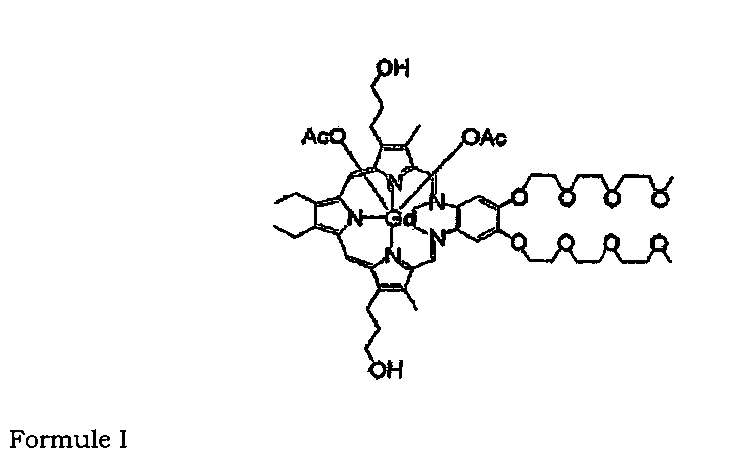 pour préparer un médicament utile pour retarder/ralentir la progression neurologique de manière sélective chez un être humain atteint d'un cancer des poumons et de métastases cérébrales dudit cancer des poumons.

2. Utilisation d'un composé de formule I pour préparer un médicament utile pour prolonger/améliorer la durée jusqu'à la progression neurologique de manière sélective chez un être humain atteint d'un cancer des poumons et de métastases cérébrales dudit cancer des poumons.

3. Utilisation d'un composé de formule I pour préparer un médicament utile pour prolonger la durée jusqu'à la détérioration de la fonction de la mémoire de manière sélective chez un être humain atteint d'un cancer des poumons et de métastases cérébrales dudit cancer des poumons.

4. Utilisation d'un composé de formule I pour préparer un médicament utile pour prolonger la durée jusqu'à la détérioration de la fonction exécutive de manière sélective chez un être humain atteint d'un cancer des poumons et de métastases cérébrales dudit cancer des poumons.

5. Utilisation selon l'une quelconque des revendications 1 à 4, dans laquelle le médicament se présente sous la forme d'une composition injectable comprenant un véhicule pharmaceutiquement acceptable.

6. Utilisation selon l'une quelconque des revendications 1 à 5, dans laquelle le cancer des poumons s'est métastasé dans le cerveau de l'être humain.

7. Utilisation selon la revendication 6, dans laquelle l'être humain est en outre soumis à une radiothérapie.

8. Utilisation selon la revendication 7, dans laquelle la radiothérapie est une radiothérapie totale du cerveau.
